Europäisches Patentamt

European Patent Office

Office européen des brevets

(19)

(11) Publication number: **0 237 002**
**A1**

# EUROPEAN PATENT APPLICATION

(12)

(21) Application number: **87103316.3**

(22) Date of filing: **09.03.87**

(51) Int. Cl.⁴: **C12N 1/20 , C12N 1/32**

(30) Priority: **10.03.86 US 838077**

(43) Date of publication of application:
**16.09.87 Bulletin 87/38**

(84) Designated Contracting States:
**AT BE CH DE ES FR GB IT LI LU NL SE**

(71) Applicant: **PHILLIPS PETROLEUM COMPANY**
**5th and Keeler**
**Bartlesville Oklahoma 74004(US)**

(72) Inventor: **Shay, Lucas Kingyuang**
**617 SE Castle Rd.**
**Bartlesville Oklahoma 74006(US)**
Inventor: **Wegner, Eugene Herman**
**618 SE Brookhollow Ln.**
**Bartlesville Oklahoma 74006(US)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) **Fermentation of bacteria at high productivities.**

(57) A method is disclosed for high productivity bacterial fermentation employing high mineral salts feed to the aqueous ferment. For example, single cell protein (SCP) is produced from *Methylomonas sp.* in an aerobic bacterial fermentation process at high yields under high productivity conditions employing a media containing a high mineral salts concentration.

EP 0 237 002 A1

## FERMENTATION OF BACTERIA AT HIGH PRODUCTIVITIES

The invention relates to high productivity processes for the production of single cell protein by bacteria.

## BACKGROUND OF THE INVENTION

Much effort has been expended to develop various fermentation processes in which single cell protein - (SCP) is obtained and/or various chemical conversions are achieved by the growth of a variety of microorganisms on various carbon-containing substrates. The SCP product is useful as a protein food-source, while chemical conversions offer sources of new chemicals such as bio-polymers, pharmaceuticals, and enzymes, even commodity chemicals. In addition, genetically modified organisms can be employed to produce valuable polypeptide products.

The carbon energy substrates employed for the growth of microorganisms should be relatively cheap, readily available, and preferably water-soluble. Initially, hydrocarbons were considered as carbon energy sources for SCP. However, oxygenated hydrocarbons, such as alcohols and sugars, are more preferred as sole or partial substrates due to their relative water-solubility in an aqueous ferment, and in the reduced molecular oxygen requirements for microbial conversion-growth processes.

A limiting factor in efforts to commercialize various fermentation processes has been the inability to maintain the aqueous ferment at greater than modest cell densities, i.e., cell densities in excess of about 40 grams per liter of aqueous ferment (grams of washed, dried cells per liter of cells plus liquor). This has generally required handling large volumes of fermentation liquor effluent in order to recover moderate amounts of SCP or other product. Handling large volumes of fermentation liquor effluent complicates the concentration of the SCP or other products by requiring the use of equipment such as centrifuges; as well as by requiring washing, drying and other recovery steps.

Methods have recently been disclosed to grow yeasts at cell densities of up to about 150g/liter. See U.S. 4,414,329 (Wegner, Nov. 8, 1983). This breakthrough has made practicable for the first time recovery of SCP from an aqueous ferment without the need for intervening cell concentration step. In addition, the Wegner process has made possible the production of higher concentrations of valuable products in the aqueous ferment with easier product recovery. Both of these advantages are achieved with the added benefits of much reduced volumes of liquid-handling, and much lower power consumptions and lower equipment sizing costs.

In spite of the success of Wegner in the field of yeast fermentation, corresponding efforts to achieve higher cellular densities with bacteria have been fruitless thus far.

Bacteria have several advantages over yeast as a source of SCP. They grow faster, have higher protein content, are often more tolerant to higher fermentation temperatures (which are more economical to run under), require no external growth factors, e.g. vitamins, and have generally higher yields. Bacteria are a better source of nucleic acids than yeasts having approximately twice the nucleic acids content. Nucleic acids find uses as flavor enhancers and as chemical building blocks in the chemical and pharmaceutical industries.

Many bacteria are of industrial importance because they produce a variety of enzymes, e.g., amylases, and chemicals, e.g., amino acids and antibiotics. In addition, application of recombinant DNA techniques has made some species of bacteria, such as, for example, *Escherichia* and *Bacillus* species, capable of producing valuable enzymes, chemicals and polypeptides. Unfortunately, however, because conventional fermentation processes are only capable of growth to cell densities of about 30-40 g/L, the productivity of the desired products is quite limited. Therefore, an improvement in the productivity, i.e., grams of cells produced per liter of broth per hour (g/L/hr), upon fermentation of carbon-containing substrates, would be highly desirable.

## OBJECTS OF THE INVENTION

An object of the present invention, therefore, is a process for the production of bacterial cells at high productivity.

Another object of the present invention is a process for the efficient chemical conversion of carbon-containing substrates into high value fermentation products.

2

These and other objects of the invention will become apparent from inspection of the detailed description and appended claims provided herewith.

## STATEMENT OF THE INVENTION

In accordance with the present invention, we have discovered a fermentation process to grow bacteria in bacterial fermentations at high cellular productivities. While bacteria have not previously been amenable to high productivity fermentation, we have found, unexpectedly, that by providing a specific balance of high salts feed to the aqueous ferment inoculated with a bacterium, a high productivity of the bacterial cells is obtained in the aqueous ferment.

This invention is of tremendous importance and represents a true breakthrough since genetically modified bacteria, e.g., *E. Coli* and *Bacillus sp.* have become so important in the commercialization of recombinant DNA products.

The high salts/high productivity method of the present invention is effective to achieve high cell densities in the fermentor without requiring physical means of cellular build-up. Such physical means include removing aqueous ferment and separating cells for recycle to the fermentor, or using an internal separator in the fermentor to separate out liquor for removal in order to build up cell counts. Such old methods are expensive, difficult, at times harmful to the cells, lack adequate control, and add markedly to the apparatus and power costs.

Heretofore, continuous processes for the production of single cell protein materials from bacterial cultures typically gave ferment effluents of relatively low bacterial cell contents, such as about 20 to 40 grams of cells per liter of ferment, i.e., productivities in the range of about 5 to 10 g/L/hr. Our invention, however, provides a process whereby fermentation produces bacterial cells at very high levels, i.e., productivities in excess of 10 g/L/hr and/or densities of at least 50 grams per liter of ferment are obtained. Such high productivities and cell densities in the ferment, coupled with high yields of bacterial cells, mean more efficient production of biomass and related cellular products, particularly under continuous fermentation conditions.

In accordance with our invention, employing high productivity conditions, productivities as high as 55 g/L/hr have been obtained. When retention times are maintained at a sufficiently high level, cell densities within the range of about 80 to 150, preferably about 100 to 120, grams of bacterial cells, on a dried basis, per liter of fermentation fluid, can be obtained in high yield.

For purposes of this disclosure, cell density is defined as the concentration of cells by weight on a dry basis per volume of ferment. The ferment is defined as the total ungased liquid volume of aqueous fermentation broth or liquor, including cells. Cell density usually is expressed as grams/liter.

Yield is defined as the amount of cells by weight produced for a given consumption by weight of carbon energy source or substrate fed to the fermentor, and is usually expressed as grams/gram, (i.e., grams of cells produced per gram of substrate feed). While yield is sometimes reported in terms of dried ferment per liter of aqueous ferment, preferably, yield should be reported in terms of washed dried cells per liter of aqueous ferment, which is more accurate. In our specification, yield is always reported as washed, dried cells per liter of aqueous ferment, unless otherwise specified.

## DETAILED DESCRIPTION OF THE INVENTION

In accordance with our invention, bacteria are grown at high productivities under substantially continuous aerobic aqueous fermentation conditions on a suitable non-hydrocarbon substrate, employing a suitable source of oxygen, an assimilable-nitrogen source, and a high input of defined ratios of nutrient mineral salts.

It is indeed surprising, in our experience, to achieve such high productivities, high cell densities and high yields with bacteria. The productivities achievable by the practice of our invention are as high as 55 g/L/hr, while the high cellular densities achievable by our high salts feed process are as high as about 150 g/liter of aqueous ferment.

Culturing is accomplished in a growth medium comprising an aqueous mineral salts medium, a suitable non-hydrocarbon substrate as the carbon energy source material, molecular oxygen, an assimilable-nitrogen source, and of course, a starting inoculum of one or more particular species of bacteria to be employed.

It is necessary to supply suitable amounts of selected mineral nutrients in proper proportions in the feed media in order to assure proper bacterial growth, to maximize assimilation of the carbon energy source by the cells in the microbial conversion process, to achieve maximum cellular yields with maximum productivity and cell density in the aqueous fermentation media.

Although the composition of the aqueous ferment can vary over a wide range, depending in part on the bacteria and substrate employed, in accordance with our invention, the minerals content in the ferment, i.e., liquid plus cells, is relatively high. Indeed, in the practice of the present invention, the minerals content in the ferment is maintained at higher levels than heretofore considered suitable by the prior art for bacterial fermentations. Set forth in Table I below are the minimum (i.e., broad), preferred and presently most preferred ranges of concentrations of the various elements required to be in the aqueous ferment. The concentration in each instance is expressed as of the element, though it is recognized that all or part of each can be present in the form of a soluble ion, such as where P is present in a combined form, such as the phosphate. The amount of each element is expressed in grams or milligrams of element per liter of ferment (aqueous phase, including cells):

## TABLE I

### Weight of Element per Liter of Ferment

| Element, Units | Broad Range | Preferred Range | Most Preferred |
|---|---|---|---|
| P,g | 1.3-6.5 | 2.0-5.0 | 2.5-4.0 |
| K,g | 1.0-4.0 | 1.3-3.5 | 2.0-3.0 |
| Mg,g | 0.4-1.6 | 0.5-1.3 | 0.6-1.0 |
| Ca,g | 0.1-0.7 | 0.13-0.5 | 0.2-0.3 |
| S,g | 0.5-2.6 | 0.7-2.3 | 0.7-2.1 |
| Fe,mg | 10-80 | 25-65 | 25-50 |
| Zn,mg | 5-65 | 10-50 | 20-40 |
| Cu,mg | 1-5 | 1.5-3 | 1.7-2.6 |
| Mn,mg | 1-65 | 2.6-40 | 4-15 |
| Mo,mg | 0.2-10 | 0.5-8 | 0.8-3 |
| Co,mg | 0.1-0.7 | 0.1-0.5 | 0.2-0.4 |
| B,mg | 0.05-0.4 | 0.09-0.35 | 0.1-0.26 |

Sulfur desirably is employed in the form of sulfate. Some of the metals required are advantageously added in the form of a sulfate. Thus, the minimum concentrations of sulfur normally are exceeded. Preferably, magnesium, calcium, iron, zinc, copper, manganese and cobalt are employed in the form of a sulfate, or in the form of a compound which is converted in-situ to a sulfate. Molybdenum and boron are preferably employed as the molybdate and borate, respectively. Potassium preferably is employed as a sulfate or phosphate, or in the form of a compound which is converted in-situ to a sulfate or phosphate. The phosphorus preferably is employed in the form of phosphoric acid or in the form of a phosphate, monohydrogen phosphate, or dihydrogen phosphate, e.g., as a potassium or ammonium salt, or as a compound which is converted in-situ to such a salt.

Conveniently, a primary mineral salts medium can be employed to include nutrients comprising P, K, Mg, S, and Ca; and a trace mineral medium to supply nutrients comprising Fe, Zn, Mn, Mo, Co, B and Cu.

Other elements which may be present, at least in trace amounts, include such as sodium, e.g., as a sulfate; selenium, e.g., as selenite or selenate; and iodine, e.g., as iodide.

## BACTERIA

According to our high productivity, high cell density bacterial process, we grow a culture of a bacterium on suitable carbon-containing substrates under aqueous fermentation conditions.

While bacterial genus species in general are suitable for growth in accordance with our invention, it is preferred that organisms which do not secrete significant quantities of inhibitory compounds or compounds which are detrimental to the viability of the growing cells be employed in the practice of our invention. In particular, three specific species/strains are presently preferred. These are the *Methylomonas* sp. "L3" (also referred to herein as "PL3"), deposited with the United States Department of Agriculture and assigned the accession number NRRL B-15416; the *E. Coli* strain obtainable from the American Type Culture Collection ATCC as ATCC 10798; and the *Bacillus megaterium* strain QM B1551 obtainable from the U.S. Army Laboratories in Natick, Massachusetts.

Other exemplary bacterial species include:

*B. acidocaldarius,*
*B. alcalophilus,*
*M. methylotrophus,*
*E. blattae,*
*E. intermedia,*
*Pseudomonas sp.,*
*B. subtillis,* and
*B. lioheniformis.*

The *Methylomonas Sp.* strain we refer to as "PL3" is a strain courteously supplied by Henry C. Lim, PhD. of Purdue University, designated by him as "L3" and deposited by us at the USDA-NRRL to insure availability thereof to the public upon issuance of this application as a patent. PL3 has been assigned the accession number NRRL B-15416 by the depository. This strain is described by Dr. Lim in an article by Hirt, Papoutsakis, Krug, and Lim, "Formaldehyde Incorporation by a New Methylotroph (L3)", published in Applied and Environmental Microbiology July, 1978, 56-62.

The designation NRRL B-15416 reflects the fact that the bacterial culture PL-3 (L3) has been deposited with an official depository, the United States Department of Agriculture, Agricultural Research Service, Northern Regional Research Laboratory, Peoria, Illinois 61604. The deposits have been made in accordance with the Patent and Trademark Office practice such that all restrictions on availability of the strain to the public will be irrevocably removed upon granting of a patent on this application, of which this important strain is the subject. Thus, this strain will be available to the public for utilization in accordance with our invention.

## SUBSTRATE

In our high productivity, high cell density bacterial fermentations, the carbon-containing substrate is selected from the group consisting of:

one or more of the lower aliphatic water-soluble alcohols or amines having one to four carbon atoms per molecule,

carbohydrates such as for example, simple sugars having three to six carbon atoms, disaccharides, olisosaccharides, polysaccharides, and the like. Presently preferred is methanol for *Methylomonas Sp.* and carbohydrates such as glucose and sucrose for *Escherichia* and *Bacillus* sp.

## FERMENTATION CONDITIONS

In our process, mineral salts are added at relatively high levels in the aqueous liquor, resulting in a continuous fermentation process at relatively high productivities, producing high bacterial cell densities, with high yields of bacterial cells.

5

In our high cell density bacterial fermentation, the aqueous ferment comprises about one-half supernatant, i.e., cell-free liquid, and one-half bacterial cells, by volume. These one-half by volume bacteria cells, however, will contain at least about two-thirds of the mineral salts content of the ferment.

The salts in the supernatant are at a relatively low concentration, since there is a high take-up by the growing reproducing cells. The mineral salts in the cells may not be as fed or applied since some may be in a bound organic form. Mineral analysis of the aqueous ferment, of course, would reflect a total mineral content.

The fermentation is an aerobic process requiring molecular oxygen which is supplied by a molecular oxygen-containing gas such as air, oxygen-enriched air, or substantially pure molecular oxygen, so as to maintain the ferment with an oxygen partial pressure effective to assist the bacteria in growing and in biochemically converting substrate in a thriving fashion.

The rate at which molecular oxygen is fed to the ferment should be such that the growth of the bacteria is not limited by lack of oxygen. Fermentor designs vary widely in their ability to transfer oxygen to the culture. Preferably, fermentation apparatus capable of providing an oxygen transfer rate of at least about 600 mmol $O_2$/L/hr will be employed, to ensure that lack of oxygen is not a problem when cells are grown to high productivity and/or high cell density.

The type of fermentor employed in the practice of our invention is not critical, though presently preferred is operation in a foam-filled fermentor. A fermentor designed to encourage and maintain the produced foam usually is beneficial to the process of achieving the increased oxygen transfer necessary to maintain desired high cell densities and rapid growth rates.

Although the overall aeration rates can vary over a considerable range, with fermentors that are efficient in oxygen transfer, aeration generally is conducted at a rate of about 0.5 to 8, preferably about 1 to 6, volumes (at the pressure employed and at 25°C) of molecular oxygen-containing gas per liquid volume in the fermentor per minute. This amount is based on air of normal oxygen content being supplied to the reactor, so that in terms of pure molecular oxygen the respective ranges would be about 0.1 to 1.7, or preferably about 0.2 to 1.3, volumes (at the pressure employed and at 25°C) of molecular oxygen per liquid volume in the fermentor per minute.

The pressure employed for the microbial fermentation step can range widely. Typical pressures employed are about 0 to 150 psig, presently pressures in the range of about 0 to 60 psig are preferred, more preferably pressures in the range of 35 to 40 psig are employed. At such preferred pressures a reasonable balance of equipment and operating costs versus oxygen solubility is achieved. Greater than atmospheric pressures are advantageous in that increased dissolved oxygen concentration in the aqueous ferment is obtained, which in turn increases cellular growth rates. This is counterbalanced, however, by the fact that high pressures increase equipment and operating costs.

The fermentation temperature can vary somewhat, but generally will be in the range of about 25°C to 80°C, presently preferred about 30 to 60°C. The most preferred temperature for a given bacterium can be readily determined by one of skill in the art.

Bacteria require a source of assimilable nitrogen. The assimilable nitrogen can be supplied by any nitrogen-containing compound or compounds capable of releasing nitrogen in a form suitable for metabolic utilization by the microorganism. While a variety of organic nitrogen source compounds, such as protein hydrolysates, technically can be employed, usually cheaper nitrogen-containing compounds such as ammonia, ammonium hydroxide, urea, and various ammonium salts such as ammonium phosphate, ammonium sulfate, ammonium pyrophosphate, and ammonium chloride can be utilized. Ammonia gas itself is convenient for large scale operations, and can be employed by bubbling through the aqueous microbial ferment in suitable amounts. At the same time, ammonia can be used to assist in pH control.

The pH range in the aqueous microbial ferment for bacteria should generally be maintained in the range of about 5 to 8, preferably about 6 to 7.5. Preferences of certain bacteria for a pH range are dependent to some extent on the medium employed, as well as on the particular bacteria, and thus may change somewhat with change in medium as can be readily determined by those skilled in the art.

The average retention time of the aqueous ferment in the fermentor can vary considerably, depending in part on the fermentation temperature, oxygen availability, and the bacterial culture employed. Generally, the retention time will vary in the range of about 1 to 20 hours, presently preferably about 2 to 8 hours, based on average retention.

High concentrations of certain substrates may be inhibitory to satisfactory bacterial growth, or even toxic to the microorganisms in the fermentation. Relatively high concentrations of such substrates as methanol thus should be avoided, so that it is generally desirable to maintain the methanol concentration in the ferment at a maximum tolerable level. This level in the ferment generally is within the range of about 0.005 to 1 volume percent, preferably about 0.003 to 0.01 volume percent.

6

Conveniently, the fermentation is conducted in such a manner that the substrate is controlled as a limiting factor, providing good conversion of the substrate to bacterial cells and extracellular products, and avoiding loss of unconverted substrate.

Continuous operation is preferred for ease of control, production of uniform quantities of cells or extracellular products, and most economical uses of all equipment. In a continuous process, the substrate, aqueous mineral medium, assimilable nitrogen source, and molecular oxygen-containing gases are added continuously to the ferment in the fermentor combined with continuous withdrawal of ferment. Although the volume ratio of added carbon energy substrate to aqueous mineral medium can vary over a wide range, depending in part on the nature of the carbon-containing substrate, generally it will be in the range of about 1:9 to 6:4, presently and preferably in the range of about 2:8 to 5:5.

One skilled in the art readily recognizes that the maximum bacterial cell density obtainable is a function of the cell yield (g of cells per g of substrate feed) and the percent substrate in the total feed to the fermentor. Total feed is the carbon substrate plus mineral media including water.

If desired, part or all of the substrate and/or part of the assimilable nitrogen source such as ammonia can be added to the aqueous mineral medium prior to passing the aqueous mineral medium to the fermentor. Most convenient in our work in high cell density bacterial fermentations has been the use of a feed containing about 30 volume percent substrate to 70 volume percent mineral salts medium.

Each of the streams introduced into the reactor preferably is controlled at a predetermined rate, or in response to a need determinable by monitoring, such as concentration in the ferment of the carbon and energy substrate, the pH, the dissolved oxygen, the cell density measurable by light transmittancy, or the like, and the oxygen or carbon dioxide in the off-gases from the fermentor. The feed rates of the various materials streams can be varied so as to obtain as rapid a cell growth rate as possible, consistent with efficient utilization of the carbon and energy source, to obtain as high a yield of bacterial cells and/or extracellular products, relative to substrate charge as possible, or to maximize the particular biochemical conversion being practiced. Thus, by the process of our invention, bacterial cells can be obtained in yields of about 45 to 55 grams per 100 of grams substrate charged, depending in part on the growth conditions employed and the particular substrate used.

All fermentation equipment, e.g., reactor, vessel or container, piping, attendant circulating or cooling devices, and the like, generally are sterilized, usually by employing steam such as at about 250°F (121°C) for at least about 15 minutes. The sterilized reactor is inoculated with a culture of the specified microorganism in the presence of all the required nutrients, including molecular oxygen, and the substrate.

In starting out a continuous fermentation, the aqueous mineral medium, suitable concentration of substrate, assimilable nitrogen source, and trace components, where desired, are sterilized. These feed materials can be heat sterilized or sterilized by filtration through a series of filters, e.g., a 0.7μ, 0.45μ and 0.2μ set of filters. The sterilized materials and the starting inoculum of the desired bacterial strain are placed in a sterilized fermentor, and suitable flows of oxygen and the various feeds are gradually commenced. It is possible to begin at low mineral salts levels in the aqueous ferment and build up to a high mineral salts level by feeding an aqueous mineral medium having a high concentration of mineral salts to the ferment, though we normally simply add high salts medium initially to the fermentor to commence immediate operation. One skilled in the art realizes that a brief lag time will usually occur at start up before the inoculum builds up enough cells for full input of salts and substrate to be effectively utilized.

## PRODUCT RECOVERY

The bacterial cells produced in accordance with our high cell density process can be recovered from the fermentor by conventional means, such as by flocculation, foam concentration, centrifugation or filtration.

The microbial cells can be killed, if desired, by heat or chemical means, before or after the separation of the cells from the fermentor effluent.

If desired, the concentrated cells then can be washed such as by mixing with water, and separated such as by recentrifuging, or by adding water prior to or during centrifugation to substantially free the cells of mineral medium, and the washings including the separated mineral medium then can be returned to the fermentor as water and mineral medium makeup, thus substantially reducing or avoiding waste disposal problems.

The recovered cells can be dried to produce a dried product for future use. If desired, the high cell density fermentor effluent in total can be dried to produce a whole dried product of dried bacterial cells and residual water soluble substances including salts, and this whole-dried product used as an animal feed of high protein-high salts character. For human consumption, the cells can be treated as necessary to reduce the nucleic acid, but for animal feed purposes such treatment is not presently believed to be necessary.

Extracellular products can be recovered from the substantially cell-free supernatant liquid by conventional means. The substantially cell-free supernatant can be treated, for example, with acetone or a lower alcohol such as methanol or ethanol to precipitate extracellular polymeric material. The cell-free effluent also can be treated by solvent extraction and/or base extraction to recover, if desired, other extracellular products such as pigments, vitamins, or organic acids produced during the culturing process. The cell-free effluent, with or without such intervening treatment, can be returned to the fermentor as a part of the aqueous makeup, or as a substantial or almost total part of the aqueous makeup, to avoid waste disposal problems insofar as possible.

The high cell densities obtainable by our process significantly streamline and reduce the cost of single cell protein production from bacteria. The need to concentrate the resulting single cells for use as a protein product in many instances can be sharply reduced or even eliminated. The cellular product can, if desired, be washed to remove residual unconsumed salts and extracellular products such as amino acids, biopolymers, extracellular enzymes, and the like. The washed cells can then be sent directly to a dryer means such as a spray dryer.

Requirements for water to the fermentation step thus are reduced considerably, and, importantly, there is little or no waste water requiring disposal. Alternatively, if desired, the total ferment including residual salts can be dried.

The following examples are provided in an effort to assist one skilled in the art to a further understanding of our invention, and yet not to be unduly limitative of the reasonable scope of our invention. The particular reactants, conditions, ratios, and the like, are all intended to be illustrative of our invention, and not limitative of the reasonable and suitable scope thereof.

### Example I

A gram-negative methanol-utilizing bacterium L3 was obtained from Purdue University. The bacterium L3 was grown in 100 mL of IM2 medium containing 1.0 percent methanol in a shake flask maintained at 30°C.

### IM2 Recipe

| | |
|---|---|
| $H_2O$ | 1.0 L |
| $KH_2PO_4$ | 2.0 g |
| $K_2HPO_4$ | 3.0 g |
| $MgSO_4 \cdot 7H_2O$ | 0.4 g |
| $CaCL_2 \cdot 2H_2O$ | 0.4 g |
| NaCl | 0.1 g |
| $(NH_4)_2SO_4$ | 2.0 g |
| trace minerals | 0.05 mL |

The trace mineral solution was prepared by mixing, for each liter of solution:

| | g/L |
|---|---|
| $CuSO_4 \cdot 5H_2O$ | 5.0 |
| KI | 0.8 |
| $Na_2MoO_4 \cdot 2H_2O$ | 2.0 |
| $H_3BO_3$ | 0.5 |
| $ZnSO_4 \cdot 7H_2O$ | 23.0 |
| $CoCl_2 \cdot 6H_2O$ | 0.5 |
| $FeSO_4 \cdot 7H_2O$ | 80.5 |
| $MnSO_4 \cdot H_2O$ | 9.5 |

The next day, the overnight culture was inoculated in a 4 L fermentor containing 2 L of FM12 medium with 4.5 percent methanol as carbon and energy source.

### FM12 Recipe

| | |
|---|---|
| $H_2O$ | 1.0 L |
| $H_3PO_4$ (85%) | 2.0 mL |
| KCl | 1.0 g |
| $MgSO_4 \cdot 7H_2O$ | 1.5 g |
| $CaCl_2 \cdot 2H_2O$ | 0.2 g |
| NaCl | 0.1 g |
| trace minerals | 0.5 mL |

Appropriate aeration was applied to the fermentor. When the initial methanol charge was consumed, FM12 containing 5 percent methanol was fed continuously to the fermentor. The reactor volume was maintained at about 2 L. At a dilution rate of 0.24 $h^{-1}$ or a 4.2 h retention time, the cell mass yield was very high (53 percent). The cell density was 21 grams per liter, and cell mass productivity was about 5 g/L/hr.

### Example II

Bacterium L3 in a 4L fermentor was continuously fed with two feed streams, wherein 70% of the total volume fed was mineral media PL3 and 30% of the total volume fed was methanol.

PL-3 medium had the following composition per liter:

| | |
|---|---|
| 75% $H_3PO_4$ | 16.4 mL |
| $CaSO_4 \cdot 2H_2O$ | 1.5 g |
| $K_2SO_4$ | 4.5 g |
| $MgSO_4 \cdot 7H_2O$ | 8.8 g |

The dry weight of total fermentor effluent, i.e., cells plus soluble effluent components, was 134 g/L and the cell mass was 114 g/L, or 48.6 percent yield, with a productivity of about 28.5 g/L/hr.

At such high cell densities as achieved herein, the fermentor effluent can be direct-dried. In addition to eliminating recovery loss, such direct-dried process can also eliminate the need for certain recovery equipment and contamination occurred during recovery.

### Example III

A 4 liter fermentor equipped for continuous operation with an air sparging plate, stirrer, dissolved oxygen probe, pH probe, temperature measuring means and suitable inlets for adding media, ammonia and substrate (glucose) and an outlet for withdrawing effluent was charged with one liter of inoculum of the bacterium *Escherichia coli*, strain K-12, obtained from American Type Culture Collection (ATCC) Rockville, Maryland under the deposit number ATCC 10798. The aqueous media utilized for the run was PL3 media which formulation was set forth in Example II.

Ammonia was utilized to provide pH control as well as an assimilable source of nitrogen for the microbe.

The carbon energy source (substrate) was glucose and was charged during the run as an aqueous solution of about 50 percent wt/vol glucose.

The fermentor controls were set to operate at a pH of about 6.5 and at a temperature of about 38°C. The stirrer operated at 1000 rpm.

Samples of fermentor effluent were periodically removed for analysis. A portion of the sample (20 mL) was evaporated to dryness to determine total solids in g/L. Another portion (80-100 mL) was centrifuged and a portion (20 mL) of the supernatant evaporated to dryness to determine solids content of the supernatant. The centrifuged solid phase was resuspended in 20-25 mL tap water and centrifuged again. The resulting solid phase was dried to provide the weight of washed cells in g/L of fermentor effluent.

Table II below shows operating conditions at the times indicated and the corresponding analysis results on the fermentor effluent.

The fermentation run was terminated after 188 hours, although the run could have been continued for a longer period, if desired.

## TABLE II

| Time After Inoculation hrs | Retention Time, hrs | Glucose concn. wt. % | Wt. of Solids, g/L | | | Yield,[b] % | Productivity g/L/hr. |
|---|---|---|---|---|---|---|---|
| | | | Total | Washed Cells | Supernatant | | |
| 92 | 10.8 | 24.7 | 101.7 | 92.0 | 24.5 | 37.3 | 8.5 |
| 116 | 2.98 | 20.4 | 89.3 | 72.1 | 24.7 | 35.3 | 24.2 |
| 140[a] | 2.42 | 20.2 | 104.2 | 59.3 | 32.2 | 29.4 | 24.7 |
| 164 | 2.7 | 25.3 | 107.1 | 110.5 | 25.0 | 43.7 | 40.9 |
| 188 | 1.8 | 25.4 | 103.9 | 98.9 | 24.5 | 38.9 | 55.0 |

[a] Fermentor contents transferred to another clean sterilized fermentor because of sparger plugging problems.

[b] Based on weight of dried washed cells and weight of glucose calculated for ferment volume.

The results show that very high productivities and cell densities of *E. coli* were obtained during the course of this continuous fermentation run.

Example IV

A fermentor equipped as described in Example III was charged with an inoculum of 100 mL *Bacillus megaterium* culture grown in IM-2 media (see Example I) containing 0.5 weight percent glucose. The fermentor had previously been charged with 1 liter of PL3 media containing 20 weight percent sucrose for use during the initial stages of the continuous culture run. From about 2 hours to about 26 hours into the run the substrate used was "dextrose greens," a by-product of dextrose production by starch hydrolysis.

Ammonia was utilized to provide pH control as well as an assimilable source of nitrogen for the microorganism. The fermentor controls were set to operate at a pH of about 6.5 and at a temperature of about 34°C. The stirrer operated initially at 350 rpm, was raised to 750 and then 1000 rpm during the latter stages of the run.

At about 26 hours into the continuous run the media feed was switched to PL-3 media containing 25 percent by weight sucrose.

As in Example VI samples of fermentor effluent were periodically removed for analysis. At a retention time of about 7 hours the fermentation achieved a cell density of 111 grams per liter of washed cells and a yield of 44 percent based on carbon substrate, for a productivity of 16.1 grams per liter per hour. These values were obtained 74 hours into the run. At 90 hours into the run similar results were obtained, 110 grams per liter of washed cells, 44 percent yield, and a productivity of 15.9 grams per liter per hour.

The above results show that a bacillus species, *Bacillus megaterium* (strain QM B1551) could be grown to high cell density in a continuous fermentation process utilizing sucrose as a fermentation substrate. Strain QM B1551 was obtained from U.S. Army Laboratories, Natick, Massachusetts. Because *B. megaterium* QM B1551 produces extracellular material (capsul) that increases the viscosity of the fermentation broth, the mass transfer was greatly reduced as fermentation proceeded. With a conventional fermentation process, i.e., a non-high productivity process, productivities even half the level demonstrated herein would be difficult to achieve.

Example V

In various runs with the bacterium L3 (NRRL B-15416), we have studied the effect on the maximum cell densities obtainable of the volume percent carbon source in the feed relative to the aqueous mineral media in the total fermentor feed. From these studies, we have developed the following relationships, tabulated below for a specific substrate, methanol:

## TABLE III

| Relative Volume % Methanol in Total Feed | Expected Cell Density[1] | Yield[2] | Actual Recovered Weight (total solids) |
|---|---|---|---|
| 5 | 21.2 | 0.54 | 24.6 |
| 10 | 40.4 | 0.51 | 44.5 |
| 15 | 56.3 | 0.48 | 65.0 |
| 20 | 73.9 | 0.47 | 83.4 |
| 25 | 89.2 | 0.45 | 106.8 |
| 30 | 107.5 | 0.45 | 128.3 |
| 34 | 113.4 | 0.44 | 131.6 |

[1]The expected cell density is a calculated value obtained by multiplying the number of grams of methanol fed per liter of feed times the yield.

[2]gm Single cell product/gm MeOH feed.

In order to accommodate the increased substrate feed, the concentration of mineral medium feed preferably is adjusted. Several factors are to be taken into account, such as the increased number of cells growing at higher cell densities and the reduced volume of water employed for the mineral feed. Thus, as shown below in Table IV, the mineral medium feed that is employed for growth on 40 volume percent methanol feed may be about 6 times as concentrated as the mineral medium feed employed for growth on 10 volume percent methanol feed. On further increase of methanol feed to 50 volume percent, the mineral medium composition preferably is adjusted to about 1.5 times the concentration employed with 40 volume percent methanol feed.

## TABLE IV

### Mineral Medium Adjustment As A Function Of Methanol Feed

| Volume % Methanol in Feed | Mineral Medium Concentration[3] | Mineral Concentration Increase per 10% Increase in MeOH Feed |
|---|---|---|
| 10 | 1.0 | - |
| 20 | 2.25 | 2.25 |
| 30 | 3.86 | 1.71 |
| 40 | 6.00 | 1.56 |
| 50 | 9.01 | 1.50 |

[3]Relative to 10% MeOH feed.

Simply put, if one considers as a constant the total volume of total feed (substrate such as methanol plus aqueous mineral media), it is apparent that as the substrate amount is increased, the minerals fed must also be increased.

The preceding examples have been provided merely to illustrate the practice of our invention and should not be read so as to limit the scope of our invention or the appended claims in any way. Reasonable variations and modifications, not departing from the essence and spirit of our invention, are contemplated to be within the scope of patent protection desired and sought.

## Claims

1. A continuous fermentation process for producing bacterial cells at a productivity of at least 10 g/L/hr and for the biochemical conversion of a carbon energy substrate to products of fermentation **characterized by** culturing at least one bacterium under aerobic aqueous fermentation conditions, in an aqueous ferment comprising a cellular phase and an aqueous extracellular phase, in fermentation means free from physical means to remove liquid medium from said aqueous ferment without removing cells, in the presence of assimilable carbon energy substrate, assimilable nitrogen source, water, molecular oxygen, and mineral salts;

wherein said mineral salts are fed to the aqueous ferment in amounts sufficient to maintain in said aqueous ferment the following elements in at least the designated weights per liter of aqueous ferment: P 1.3 g, K 1.0 g, Mg 0.4 g, Ca 0.1 g, S 0.5 g, Fe 10 mg, Zn 5 mg, Cu 1 mg, Mn 1 mg, Co 0.1 mg, and Mo 0.2 mg, and B 0.5 mg.

2. The process of claim 1 characterized in that said bacterium is selected from the genera Bacillus, Escherichia, Methylomonas and Pseudomonas; in particular wherein the bacterium is Methylomonas sp. NRRL B-15416.

3. The process of any of the preceding claims characterized in that the mineral salts are maintained in the following amounts in each liter of said aqueous ferment: P 1.3 to 6.5 g, K 1.0 to 4.0 g Mg 0.4 to 1.6 g, Ca 0.1 to 0.7 g, S 0.5 to 2.6 g, Fe 10 to 80 mg, Zn 5 to 65 mg, Cu 1 to 5 mg, Mn 1 to 65 mg, Co 0.1 to 0.7 mg, Mo 0.2 to 10 mg, and B 0.05 to 0.4 mg; in particular wherein said mineral salts comprise a primary mineral salts medium and a trace mineral salts medium, and wherein said primary mineral salts medium supplies said P, K, Mg, S, and Ca; and said trace mineral salts medium furnishes said Fe, Zn, Mg, B, and Cu, Co, and Mo; in particular further employing in said aqueous ferment at least one element selected from sodium, iodine, and selenium.

4. The process of any of the preceding claims characterized in that said aqueous fermentation conditions include fermentation temperature in the range of 25 to 80°C, pH in the range of 5 to 8, pressure in the range of 0 to 1.04 MPa (gauge) and fermentation time in the range of 1 to 20 hours based on average retention; in particular wherein the cell density is maintained in the range of 80 to 150 grams, on a dried basis, per liter of aqueous ferment; in particular wherein a cellular yield of 39 to 55 grams per 100 grams substrate charged in maintained.

5. The process of any of the preceding claims characterized in that said carbon energy substrate is selected from alcohols of 1 to 4 carbon atoms and carbohydrates; in particular wherein said alcohol is methanol; in particular wherein said bacterium is Methylomonas NRRL B-15416.

6. The process of any of the preceding claims characterized by further comprising the steps of:

(a) removing a stream of said aqueous ferment from said fermentation means,

(b) separating said stream of aqueous ferment into a cell-containing fraction, and a separated aqueous media frac tion containing residual dissolved minerals,

(c) water-washing said cell-containing fraction, thereby substantially separating traces of said mineral salts from the cells, leaving a wet cell mass,

(d) drying said wet cell mass to produce dried cells, and

(e) recycling the water-washings and separated aqueous media fraction to said culturing to provide therein at least in part the makeup water and said mineral salts.

7. The process of any of claims 1 to 5 characterized by further comprising the steps of:

removing a portion of said aqueous ferment as fermentor effluent containing both a cellular phase and an aqueous extracellular phase including residual mineral salts, and drying said aqueous ferment effluent containing said cellular phase and said aqueous phase, thereby producing a dried cellular product containing residual water-soluble substances including salts.

8. The process of any of claims 1 to 5 characterized by further comprising the steps of:

(a) removing a stream of said aqueous ferment effluent from said fermentation means,

(b) centrifuging said removed aqueous ferment, thereby producing a stream of concentrated cells, and a stream of lean recycle mineral salts aqueous liquor,

(c) water-washing said concentrated cells to produce a stream of water-washings containing further residual mineral salts, and a stream of washed cells,

(d) drying said washed cells, and

(e) recycling at least a part of at least one of said lean recycle liquor and said water-washings to said aqueous ferment to provide therein at least a portion of makeup water and mineral salts.

9. The process of any of the preceding claims characterized in that said carbon energy substrate is selected from methanol, ethanol, glucose, and sucrose, or wherein said carbon energy substrate is selected from alcohols and amines of 1 to 4 carbon atoms per molecule, and carbohydrates.

10. A method of producing a protein material, characterized by culturing a biologically pure culture of the bacterium Methylomonas Sp. NRRL B-15416 in an aqueous medium, employing an oxygenated hydrocarbon as carbon energy substrate, aerobic aqueous fermentation conditions, mineral salts, assimilable nitrogen source, and oxygen, and recovering from the resulting single cell microorganisms a protein material; in particular wherein said oxygenated hydrocarbon is an alcohol containing 1 to 4 carbon atoms per molecule; in particular wherein said alcohol is methanol.

European Patent
Office

**EUROPEAN SEARCH REPORT**

Application number

EP 87 10 3316

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 131 220 (PHILLIPS PETROLEUM CO.) <br> * Page 3, lines 23-27; page 6, lines 30-36; page 7; page 8, lines 1-21; page 9, lines 12-21; page 12, lines 11-19; page 13, lines 31-36; page 14, lines 1-2 * | 1-5,9 | C 12 N 1/20 <br> C 12 N 1/32 |
| Y | * Page 10, lines 11-13 * | 6-8 | |
| Y | EP-A-0 017 853 (PROVESTA CORP.) <br> * Claim 7 * & US-A-4 414 329 (Cat. D) | 6-8 | |
| X | CHEMICAL ABSTRACTS, vol. 89, no. 17, 23rd October 1978, page 288, abstract no. 143027n, Columbus, Ohio, US; W. HIRT et al.: "Formaldehyde incorporation by a new methylotroph (L3)", & APPL. ENVIRON. MICROBIOL. 1978, 36(1), 56-62 <br> * Whole abstract * | 10 | **TECHNICAL FIELDS SEARCHED (Int. Cl.4)** <br><br> C 12 N |
| Y | Idem | 5,9 | |
| Y | US-A-2 942 977 (J.C. LEWIS et al.) <br> * Column 7, lines 24-30 * | 5,9 | |
| | --- -/- | | |

The present search report has been drawn up for all claims

| Place of search | Date of completion of the search | Examiner |
|---|---|---|
| THE HAGUE | 21-05-1987 | ALVAREZ-Y-ALVAREZ C. |

## DOCUMENTS CONSIDERED TO BE RELEVANT

Page   2

| Category | Citation of document with indication, where appropriate, of relevant passages | Relevant to claim | CLASSIFICATION OF THE APPLICATION (Int. Cl.4) |
|----------|--------------------------------------------------------------------------------|-------------------|--------------------------------------------------|
| Y | EP-A-O 127 581   (CIBA-GEIGY AG) * Claim 1 * | 5,9 | |

-----

TECHNICAL FIELDS
SEARCHED (Int. Cl.4)

The present search report has been drawn up for all claims

| Place of search THE HAGUE | Date of completion of the search 21-05-1987 | Examiner ALVAREZ-Y-ALVAREZ C. |
|---|---|---|